# EUROPEAN PATENT APPLICATION

(11) **EP 2 103 599 A1**
(43) Date of publication of application: **23.09.2009**
(21) Application number: 07831623.9
(22) Date of filing: 12.11.2007
(51) Int. Cl.: C07D 205/04

(54) **EFFECTIVE PRODUCTION PROCESS FOR MUGINEIC ACID COMPOUND**

(30) Priority: 14.11.2006 JP 2006307397
(71) Applicant: Suntory Holdings Limited, Kita-ku, Osaka-shi, Osaka 530-8203 (JP)
(72) Inventor: NAMBA, Kosuke, Mishima-gun Osaka 618-8503 (JP); MURATA, Yoshiko, Mishima-gun Osaka 618-8503 (JP)
(74) Representative: Vossius & Partner
(86) International application number: PCT/JP2007/071893
(87) International publication number: WO 2008/059782

(57) **Abstract**

A method for producing mugineic acids, which is represented by the following scheme: 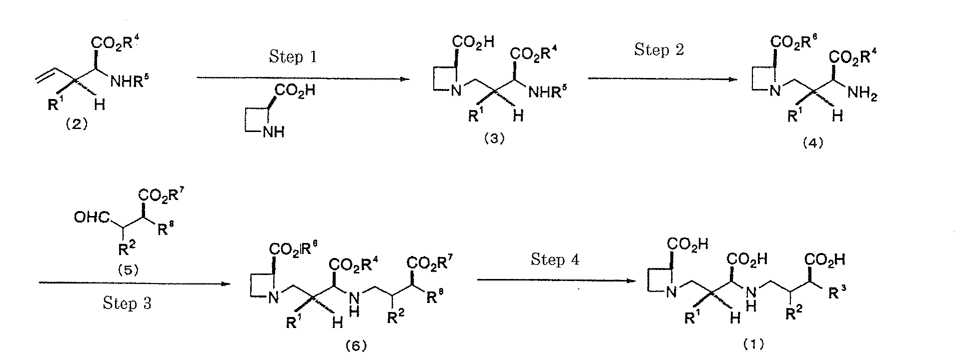 (wherein R¹ and R² represent a hydrogen atom or a hydroxyl group, R³ represents a hydroxyl group or an amino group, R⁴ and R⁷ represent a hydrogen atom or a protecting group of a carboxyl group, R⁵ represents a protecting group of an amino group, R⁶ represents a protecting group of a carboxyl group, and R⁸ represents -OR⁹ (wherein R⁹ represents a protecting group of a hydroxyl group) or -NHR¹⁰ (wherein R¹⁰ represents a protecting group of an amino group)).

### (Sequence Listing)

SEQUENCE LISTING

<110>  Suntory Limited

<120>  Manufacturing method of mugineic acid

<130>  R1717 EP S3

<140>  EP 07 83 1623.9
<141>  2007-11-12

<150>  JP2006-307397
<151>  2006-11-14

<160>  1

<210>  1
<211>  2430
<212>  DNA
<213>  Hordeum vulgare L.
<223>  HvYS1

<220>
<221>  CDS
<222>  (169)..(2202)

<400>  1
taatctcacc gcaaaagcac acggttccag ctcgcctcgt gggcggaaac ggggaacgac      60
ttcctggaag ctgcggcctc cagtgattct tcttccacgg tacagtgatc agtcgaccac     120
tacgaccttg atcgttgagc agctgcggag gcaagaagca gagccacc atg gac atc      177
                                                     Met Asp Ile
                                                     1

gtc gcc ccg gac cgc acg cgg atc gcg ccg gag atc gac agg gac gag      225
Val Ala Pro Asp Arg Thr Arg Ile Ala Pro Glu Ile Asp Arg Asp Glu
    5                   10                  15

gcc ctg gag ggc gac agg gag tcg gac ccg gcg ctg gcg tcg acg cgc      273
Ala Leu Glu Gly Asp Arg Glu Ser Asp Pro Ala Leu Ala Ser Thr Arg
20                  25                  30                  35

gag tgg cag ctg gag gac atg cca cgg tgg cag gac gag ctg acg gtg      321
Glu Trp Gln Leu Glu Asp Met Pro Arg Trp Gln Asp Glu Leu Thr Val
                40                  45                  50

cgg ggc ctg gtg gcg gcg ctg ctc atc ggg ttc atc tac acc gtc atc      369
Arg Gly Leu Val Ala Ala Leu Leu Ile Gly Phe Ile Tyr Thr Val Ile
            55                  60                  65

gtc atg aag atc gcg ctc acc acg ggg ctg gtg ccc acg ctg aac gtc      417
Val Met Lys Ile Ala Leu Thr Thr Gly Leu Val Pro Thr Leu Asn Val
        70                  75                  80

tcc gcc gcg ctg ctc tcc ttc ctg gcg ctc cgc ggg tgg acg cgc ctg      465
Ser Ala Ala Leu Leu Ser Phe Leu Ala Leu Arg Gly Trp Thr Arg Leu
    85                  90                  95

ctg gag cgc ttc ggc gtc gtg tcc cgc ccc ttc acg cgg cag gag aac      513
Leu Glu Arg Phe Gly Val Val Ser Arg Pro Phe Thr Arg Gln Glu Asn
100                 105                 110                 115

acc atc gtc cag acc tgc ggc gtg gca tgc tac acc atc gcg ttt gcc      561

Thr Ile Val Gln Thr Cys Gly Val Ala Cys Tyr Thr Ile Ala Phe Ala
                120                 125                 130

ggt ggg ttc ggg tca acc ttg ctg ggc ctg aac aag aag acg tac gag      609
Gly Gly Phe Gly Ser Thr Leu Leu Gly Leu Asn Lys Lys Thr Tyr Glu
            135                 140                 145

ctg gcc ggt gac tcg ccg ggc aac gtg ccc gga agc tgg aag gag cca      657
Leu Ala Gly Asp Ser Pro Gly Asn Val Pro Gly Ser Trp Lys Glu Pro
        150                 155                 160

ggg ata ggc tgg atg acg ggg ttc ctc ctc gct tgc agc ttc ggg ggc      705
Gly Ile Gly Trp Met Thr Gly Phe Leu Leu Ala Cys Ser Phe Gly Gly
    165                 170                 175

ctc ctc act ttg att ccc ctg aga cag gta ctg gtc gtc gac tac aaa      753
Leu Leu Thr Leu Ile Pro Leu Arg Gln Val Leu Val Val Asp Tyr Lys
180                 185                 190                 195

tta gtt tac cca agt ggg act gca act gct att ctt ata aac ggg ttc      801
Leu Val Tyr Pro Ser Gly Thr Ala Thr Ala Ile Leu Ile Asn Gly Phe
                200                 205                 210

cat acc gat caa ggg gac aag aat tca aga aag caa atc cgt gga ttc      849
His Thr Asp Gln Gly Asp Lys Asn Ser Arg Lys Gln Ile Arg Gly Phe
            215                 220                 225

ctg aaa tac ttt ggg ggt agc ttt ctg tgg agt ttc ttc caa tgg ttc      897
Leu Lys Tyr Phe Gly Gly Ser Phe Leu Trp Ser Phe Phe Gln Trp Phe
        230                 235                 240

tac act gga ggc gat gct tgt gga ttt gtt cag ttc cca act ttc ggt      945
Tyr Thr Gly Gly Asp Ala Cys Gly Phe Val Gln Phe Pro Thr Phe Gly
    245                 250                 255

ttg aag gcc tgg aag cag aca ttc tac ttt gac ttt agc atg aca tac      993
Leu Lys Ala Trp Lys Gln Thr Phe Tyr Phe Asp Phe Ser Met Thr Tyr
260                 265                 270                 275

gtt ggt gcc ggg atg att tgt cca cat ata gta aat ata tct acc ctc     1041
Val Gly Ala Gly Met Ile Cys Pro His Ile Val Asn Ile Ser Thr Leu
                280                 285                 290

ctt ggt gca att atc tca tgg gga ata atg tgg cca ctc atc agt aaa     1089
Leu Gly Ala Ile Ile Ser Trp Gly Ile Met Trp Pro Leu Ile Ser Lys
            295                 300                 305

aac aag ggg gat tgg tac cct gca aaa gta cca gaa agc agc atg aaa     1137
Asn Lys Gly Asp Trp Tyr Pro Ala Lys Val Pro Glu Ser Ser Met Lys
        310                 315                 320

agt ttg tac ggt tac aag gcc ttc ata tgc ata gct ctc atc atg ggg     1185
Ser Leu Tyr Gly Tyr Lys Ala Phe Ile Cys Ile Ala Leu Ile Met Gly
    325                 330                 335

gat ggc atg tac cac ttc ata aaa att gtt ggc atc act gct atg agc     1233
Asp Gly Met Tyr His Phe Ile Lys Ile Val Gly Ile Thr Ala Met Ser
340                 345                 350                 355

atg tat cgg caa ttt agc cac aag cag gtt aac aac aaa gca aaa aat     1281
Met Tyr Arg Gln Phe Ser His Lys Gln Val Asn Asn Lys Ala Lys Asn
                360                 365                 370

gcg gac gac act gtc tcg ctt gag gag tta cac cgc cag gag atc ttt 1329
Ala Asp Asp Thr Val Ser Leu Glu Glu Leu His Arg Gln Glu Ile Phe
            375                 380                 385

aag aga ggc cac atc ccc tct tgg atg gca tac gct ggt tat gcc ttg 1377
Lys Arg Gly His Ile Pro Ser Trp Met Ala Tyr Ala Gly Tyr Ala Leu
        390                 395                 400

ttt agc gtt ctt gca gtg gtt aca ata cca gta atg ttc aaa caa gtg 1425
Phe Ser Val Leu Ala Val Val Thr Ile Pro Val Met Phe Lys Gln Val
    405                 410                 415

aag tgg tac tat gtt gtt ata gcc tat gtc gtt gcc ccc atg ctt gga 1473
Lys Trp Tyr Tyr Val Val Ile Ala Tyr Val Val Ala Pro Met Leu Gly
420                 425                 430                 435

ttt gcc aat tca tac ggg aca ggg ctt acc gac atc aac atg ggc tat 1521
Phe Ala Asn Ser Tyr Gly Thr Gly Leu Thr Asp Ile Asn Met Gly Tyr
                440                 445                 450

aac tat ggc aag atc gct ctc ttt gtc ttt gcg gga tgg gcc ggt aaa 1569
Asn Tyr Gly Lys Ile Ala Leu Phe Val Phe Ala Gly Trp Ala Gly Lys
            455                 460                 465

gag aat ggt gtc att gcc ggc ctt gtt gct ggc acc ttg gtt aag cag 1617
Glu Asn Gly Val Ile Ala Gly Leu Val Ala Gly Thr Leu Val Lys Gln
        470                 475                 480

ttg gtg ctt atc tct gcc gat ttg atg caa gac ttc aag acg agt tac 1665
Leu Val Leu Ile Ser Ala Asp Leu Met Gln Asp Phe Lys Thr Ser Tyr
    485                 490                 495

ctc act caa aca tca cca aaa tcc atg atg att gca caa gtt gtt gga 1713
Leu Thr Gln Thr Ser Pro Lys Ser Met Met Ile Ala Gln Val Val Gly
500                 505                 510                 515

aca gcc atg ggt tgc att gtc tcc ccc ctc acg ttc atg ctc ttc tac 1761
Thr Ala Met Gly Cys Ile Val Ser Pro Leu Thr Phe Met Leu Phe Tyr
                520                 525                 530

aag gca ttt gat att ggt aac cca gat ggt act tgg aag gca cct tat 1809
Lys Ala Phe Asp Ile Gly Asn Pro Asp Gly Thr Trp Lys Ala Pro Tyr
            535                 540                 545

gca ctc ata tac cgc aat atg gca ata ctt ggt gtg gag ggc ttc tcg 1857
Ala Leu Ile Tyr Arg Asn Met Ala Ile Leu Gly Val Glu Gly Phe Ser
        550                 555                 560

gtg ttg ccg aag tat tgc atc gtg ata tcc ggt gga ttt ttc gcc ttt 1905
Val Leu Pro Lys Tyr Cys Ile Val Ile Ser Gly Gly Phe Phe Ala Phe
    565                 570                 575

gcg gcg att tta agt ata aca aga gat gtc atg ccc cac aag tat gcg 1953
Ala Ala Ile Leu Ser Ile Thr Arg Asp Val Met Pro His Lys Tyr Ala
580                 585                 590                 595

aag tat gtg ccc ctg cca atg gca atg gca gtt cca ttc ctt gta ggt 2001
Lys Tyr Val Pro Leu Pro Met Ala Met Ala Val Pro Phe Leu Val Gly
                600                 605                 610

ggg agc ttt gct att gat atg tgc ctc ggg agt ttg ata gtt ttt gca 2049
Gly Ser Phe Ala Ile Asp Met Cys Leu Gly Ser Leu Ile Val Phe Ala
            615                 620                 625

tgg acc aag ata aac aag aag gag gct ggc ttc atg gtg cct gcg gtt 2097
Trp Thr Lys Ile Asn Lys Lys Glu Ala Gly Phe Met Val Pro Ala Val
        630                 635                 640

gca tcc gct ttg ata tgt ggg gat ggc ata tgg acg ttc cct gct tcc 2145
Ala Ser Ala Leu Ile Cys Gly Asp Gly Ile Trp Thr Phe Pro Ala Ser
    645                 650                 655

att ctt gct ctt gcc aag att aaa cca cca att tgc atg aag ttt cta 2193
Ile Leu Ala Leu Ala Lys Ile Lys Pro Pro Ile Cys Met Lys Phe Leu
660                 665                 670                 675

cct gct gcc taacggaccg aaaaatatac taggtgacca aagatgcatt 2242
Pro Ala Ala

gaatttccat agttgtgcct tgctgaacta tcttttagtg tttgtttact ggagatgctt 2302
cggtactcga gtgtctagga atgatacgtt ggtatgtgct tttacgggta taacctgaat 2362
tttcttattt tatgaatctg gggtatactt atcagttgct atgagtatgg atctaatgat 2422
ttggctga 2430

## Description

### TECHNICAL FIELD

The present invention relates to an efficient method for producing mugineic acids with a reduced number of steps.

### BACKGROUND ART

Iron deficiency is presently the most prevalent human nutrition problem in the world and many peoples are suffering from the problem in both industrialized countries and developing countries. Human also ingest the necessary iron from crops. However, crops often do not absorb an adequate amount of iron from the soil. As a result, due to the lack of iron that acts as a catalyst in the formation of chlorophyll, problems such as chlorosis, low yield and a decline in nutritional value arise. Unfortunately, most crops contain only a scarce quantity of biologically available iron. In addition, the lack of available iron in soil may inhibit the growth of plant, resulting in low yield. Approximately 1/3 of the soil on earth is considered as a latent iron deficiency zone. Iron scarcity is not the sole factor that leads to the low iron intake by plants. For instance, biological availability of iron in the soil is particularly susceptible to high pH (alkalinity). Namely, the plant is unable to absorb iron adequately from the root in alkaline soil due to the existence of an iron ion in the form of water-insoluble ferric hydroxide (Fe(OH)₃). With regard to this problem, mugineic acid, a kind of metal chelating agent known as phytosiderophore secreted by graminaceous plants converts an insoluble ferric (III) into a soluble Fe-mugineic acid complex, thereby enabling iron acquisition via the uptake of such iron complex through the root (see Marschner, H. et al. , Journal of Plant Nutrition (J. Plant Nutr.), 1986, 9th volume, p.695-713: Non-Patent Document 1). Mugineic acid is a substance that chelates iron, initially isolated from iron deficient barley roots in 1978 and structurally determined (see Takemoto, T. et al., Proceedings of the Japan Academy (Proc. Japan Acad.), 1978, 54-B volume, p.469-473 : Non-Patent Document 2). Mugineic acid is a compound composed of reductively bonded azetidinecarboxylic acid unit, aspartic acid unit, and malic acid unit. Untill recent years, however, various analogs of mugineic acid (see Ma, J. F. , Nomoto, K. Physiologia Plantarum (Physiol. Plant.), 1996, 97th volume, p.609-617 : Non-Patent Document 4), such as 2'-deoxymugineic acid (see Nomoto, K. et al., Chimia, 1981, 7th volume, p.249-250 : Non-Patent Document 3) isolated from wheat have been isolated, all of which are generically referred to as mugineic acid.

Despite the great anticipation focused on further development of research or application of mugineic acids in the future, it has been very difficult to obtain these mugineic acids in large quantity. In the case of the production examples of mugineic acids, the first was the production of 2'-deoxymugineic acid reported by Ohfune et al. in 1981 (see Ohfune, Y. et al., Journal of the American Chemical Society (J. Am. Chem. Soc.), 1981, 103rd volume, p.2409-2410 : Non-Patent Document 5), concurrently an alternative method for producing 2'-deoxymugineic acid was reported by Fushiya et al. (see Fushiya, S. et al., Chemistry Letters (Chem. Lett.), 1981, p.909-912 : Non-Patent Document 6). In 1986, a method for producing mugineic acid was reported by Hamada et al. (see Hamada, Y., Shioiri, T. The Journal of Organic Chemistry (J. Org. Chem.), 1986, 51st volume, p.5489-5490 : Non-Patent Document 7). Later, Matsuura et al. further reported a simplified and improved method for producing mugineic acid (see Matsuura, F. et al., Tetrahedron, 1993, 49th volume, p.8211-8222 : Non-Patent Document 8). Furthermore, an efficient method for producing 2'-deoxymugineic acid and nicotianamine (of which the 3"-OH group is substituted with an amino group) which is the precursor of mugineic acids was reported by Miyakoshi et al. in 2001 (see Miyakoshi, K. et al. , Tetrahedron, 2001, 57th volume, p.3355-3360 : Non-Patent Document 9). Owing to the efficient method developed by Miyakoshi et al., Hasegawa Perfume Company began to commercialize nicotianamine and hence became easily accessible by many researchers. Recently, an efficient method for producing 2'-deoxymugineic acid was reported by Singh et al. (see Singh, S. et al. , Tetrahedron Letters (Tetrahedron Lett.), 2005, 46th volume, p.1419-1421 : Non-Patent Document 10). In this method, among the three components which compose mugineic acid, namely azetidinecarboxylic acid unit, aspartic acid unit and malic acid unit, 2'-deoxymugineic acid can be synthesized without protecting the azetidinecarboxylic acid unit. Later in 2005 end of year, TRC Inc. in Canada began to market 2'-deoxymugineic acid.

### DISCLOSURE OF THE INVENTION

### PROBLEMS TO BE SOLVED BY THE INVENTION

Mugineic acids have been employed as an important tool in the study exploring the iron uptake mechanism of plant. Furthermore, by virtue of its iron chelating property, mugineic acids may offer potential in various fields, for instance as a safe metal chelating agent replacing EDTA, a health food that efficiently approaches iron deficiency in animals and plants, as well as in the field of cosmetics and fertilizer. Therefore, an improvement of a method for producing mugineic acids is essential.

All methods reported hitherto involve the isolation and purification of the intermediate product. These operations entail a lot of painstaking effort and time, thereby causing the decrease of yield.

For example, methods taught by the aforementioned Non-Patent Documents 5 to 8 involve many steps, much effort is also required for the isolation and purification of the intermediate product. Therefore, further improved method is necessary to ensure a stable supply in large quantity. Also, only a low yield of 29% of 2'-deoxymugineic acid was obtained by the method taught by the aforementioned Non-Patent Document 9. Furthermore, according to the method taught by the same document, although nicotianamine which is the precursor of mugineic acids has become easily accessible owing to commercialization, 2'-deoxymugineic acid remains expensive and high yield has yet to be achieved, therefore procurement in large quantity remains difficult.

If an inexpensive supply of mugineic acids in large quantity becomes available, there will be innumerable contributions to respective researches and application possibility in fields ranging from health food to cosmetics and fertilizer. However, in the methods reported hitherto as described above, there have been problems in large quantity supply at low cost due to many steps, effort required for the isolation and purification of the intermediate product, low yield and the like.

The present invention has been made to solve the above problems of the prior art and an object thereof is to provide a method for producing mugineic acids in large quantity at low cost. Namely, in order to produce mugineic acids in practice, indispensable tasks include: 1) reduction of the number of steps involved; 2) omission of an isolation and purification step of an intermediate product, 3) simplification of production operation and 4) inexpensive reaction reagent. Thus, an object of the present invention is to provide a method for producing mugineic acids that resolve all problems at one time.

### MEANS FOR SOLVING THE PROBLEMS

Mugineic acid is a compound composed of three units namely azetidinecarboxylic acid unit, aspartic acid unit and malic acid unit. During the coupling reaction of these units, desorption of a lot of protecting groups of a carboxyl, amino or hydroxyl group of these units is required. Such operation, in turn, adds to the number of steps involved. Thus, the present inventors have attempted to reduce the number of steps by restricting the use of protecting groups of a carboxyl, amino or hydroxyl to the minimum. Namely, the present inventors either do not introduce protecting group into the azetidinecarboxylic acid unit and the carboxyl group of aspartic acid unit, or develop a method which directly proceeds to the next step without isolation and purification even with the introduction of protecting group, thereby discovering a way to reduce the number of steps and to simplify the operation. Hence, the present invention has been completed upon intensive studies made by the present inventors.

Namely, the present invention relates to the followings:
(1) A method for producing mugineic acids represented by the following general formula (1): 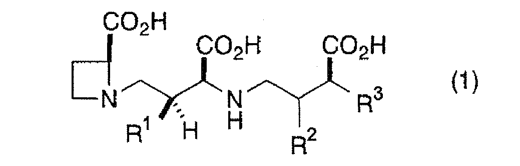 (wherein R¹ represents a hydrogen atom or a hydroxyl group, R² represents a hydrogen atom or a hydroxyl group, and R₃ represents a hydroxyl group or an amino group), which comprises:
   a step 1 of simultaneously performing oxidative cleavage of a vinyl group of a compound represented by the following general formula (2): 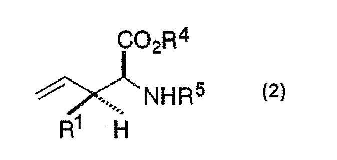 (wherein R¹ is as defined above, R⁴ represents a hydrogen atom or a protecting group of a carboxyl group, and R⁵ represents a protecting group of an amino group), and a reductive amination reaction with azetidine-2-carboxylic acid to obtain a compound represented by the following general formula (3): 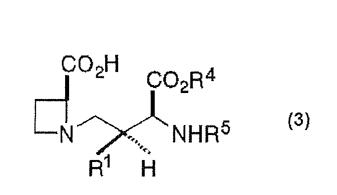 (wherein R¹, R⁴ and R⁵ are as defined above);
   a step 2 of protecting a carboxyl group of the compound represented by the general formula (3) with a protecting group and removing the protecting group of the amino group to obtain a compound represented by the following general formula (4): 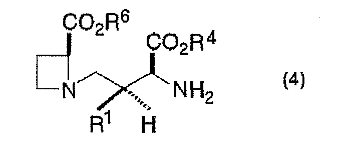 (wherein R¹ and R⁴ are as defined above, and R⁶ represents a protecting group of a carboxyl group) or their salts;
   a step 3 of subjecting an aldehyde represented by the following general formula (5): 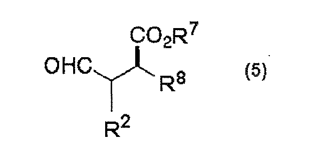 (wherein R² is as defined above, R⁷ represents a protecting group of a carboxyl group, and R⁸ represents -OR⁹ (wherein R⁹ represents a protecting group of a hydroxyl group) or -NHR¹⁰ (wherein R¹⁰ represents a protecting group of an amino group)) to an reductive amination reaction with the compound represented by the above general formula (4) to obtain a compound represented by the following general formula (6): 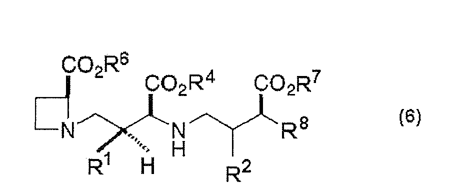 (wherein R¹, R², R⁴, R⁶, R⁷ and R⁸ are as defined above); and
   a step 4 of removing the protecting group of the carboxyl group and the protecting group of the hydroxyl group or the amino group of the compound represented by the above general formula (6);
(2) A compound represented by the following general formula (3-1): 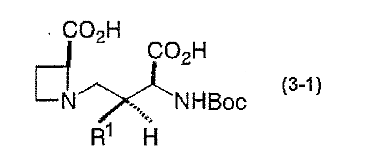 (wherein R¹ represents a hydrogen atom or a hydroxyl group, and Boc represents a tert-butoxycarbonyl group); and
(3) A compound represented by the following general formula (4-1): 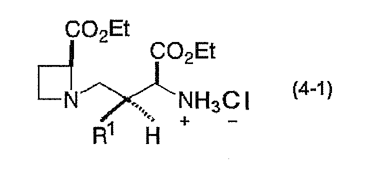 (wherein R¹ represents a hydrogen atom or a hydroxyl group, and Et represents an ethyl group).

### EFFECT OF THE INVENTION

According to the method for producing mugineic acids of the present invention, it is possible to produce mugineic acids using an inexpensive reaction reagent. Also, according to the method for producing mugineic acids of the present invention, only by sequentially adding the reaction reagents from the starting material, it is possible to obtain a protected derivative in which a functional group of a final target substance is protected with a protecting group. As a result, purification is performed only a simple and one-time operation in the entire step. Therefore, according to the present invention, mugineic acids can be easily produced within a short time. Furthermore, as a major feature of the method of the present invention, it is possible to obtain the target mugineic acids at very high yield despite most reactions are performed as one-pot reaction. The speeding up of the entire step and simplification of the production operation attributed to the method for producing mugineic acids of the present invention lead to a huge advantage in supplying mugineic acids in large quantity at low cost. According to the actual estimate of the entire production operation based on the method for producing mugineic acids of the present invention, it is possibly inexpensive in comparison with those presently on the market.

### BRIEF DESCRIPTION OF THE DRAWINGS

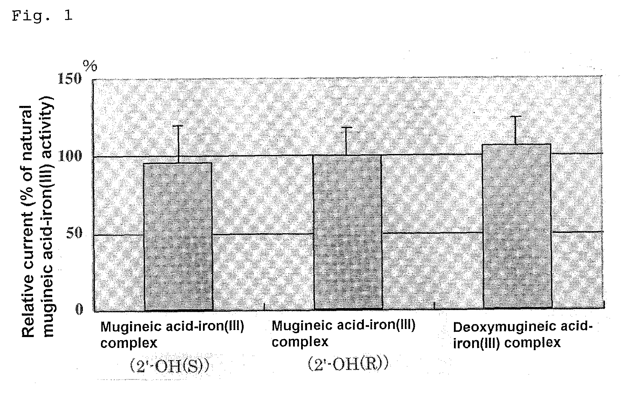

Fig. 1 shows the results of the activity of S isomer of 2'-hydroxy group of mugineic acid (natural form), R isomer of 2'-hydroxy group of mugineic acid and 2'-deoxymugine acid as a substrate of the iron complex transporter, in comparison with activity of natural mugineic acid.

### BEST MODE FOR CARRYING OUT THE INVENTION

The present invention relates to a novel method for producing mugineic acids such as mugineic acid, 2'-deoxymugineic acid, 3-hydroxymugineic acid and 3-epihydroxymugineic acid, as well as a precursor thereof such as nicotianamine or 2"-hydroxynicotianamine.

In the present invention, examples of the "protecting group of a carboxyl group" represented by R⁴, R⁶ or R⁷ include an ester residue, and examples of the ester residue include a C₁₋₆ linear, branched or cyclic lower alkyl group such as methyl, ethyl, n-propyl, isopropyl, n-butyl, isobutyl, tert-butyl, n-hexyl, or cyclohexyl; an aralkyl group such as benzyl, p-nitrobenzyl, o-nitrobenzyl, m-nitrobenzyl, 2,4-dinitrobenzyl, p-chlorobenzyl, p-bromobenzyl, or p-methoxybenzyl; and a lower aliphatic acyloxymethyl group such as acetoxymethyl, acetoxyethyl, propionyloxymethyl, n-butylyloxymethyl, isobutylyloxymethyl, or pivaloyloxymethyl. The protecting group of a carboxyl group is preferably a lower alkyl group, and particularly preferably ethyl or tert-butyl.

In the present invention, examples of the "protecting group of an amino group" represented by R⁵ or R¹⁰ include an alkoxycarbonyl group such as methoxycarbonyl, ethoxycarbonyl, 2,2,2-trichloroethoxycarbonyl, or tert-butoxycarbonyl (hereinafter abbreviated to Boc); an alkenyloxycarbonyl group such as vinyloxycarbonyl; an aralkyloxycarbonyl group such as benzyloxycarbonyl (hereinafter abbreviated to Cbz) or 9-fluorenylmethoxycarbonyl; a substituted or unsubstituted aralkyl group such as benzyl or 4-methoxybenzyl; an acyl group such as formyl, acetyl, trifluoroacetyl, or benzoyl; an arylsulfonyl group such as p-toluenesulfonyl or benzenesulfonyl; and an alkylsulfonyl group such as methanesulfonyl. The protecting group of an amino group is particularly preferably Boc or Cbz.

In the present invention, examples of the "protecting group of a hydroxyl group" represented by R⁹ include a C₁₋₆ linear or branched lower alkyl group such as methyl, ethyl, n-propyl, isopropyl, n-butyl, isobutyl, tert-butyl, or n-hexyl; a trialkylsilyl group such as trimethylsilyl, triethylsilyl, tert-butyldimethylsilyl; an acetal type protecting group such as tetrahydropyran-2-yl, methoxymethyl, or methoxyethoxymethyl; an alkoxycarbonyl group such as tert-butoxycarbonyl; and an aralkyl group such as benzyl. The protecting group of a hydroxyl group is preferably a lower alkyl group, and particularly preferably tert-butyl.

The method for producing mugineic acids of the present invention comprises the following steps 1 to 4: 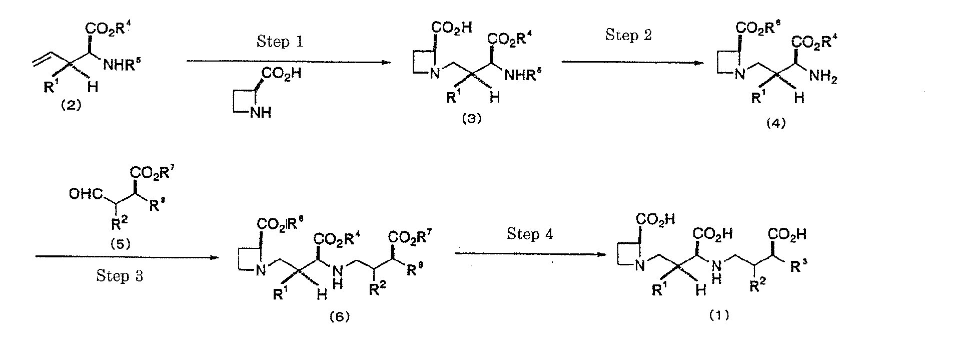 (wherein all symbols are as defined above).

The respective steps will now be described in detail.

### (1) Step 1

In the step 1, a compound represented by general formula (2) as a raw material (hereinafter abbreviated to as a compound (2)) is preferably, for example, Boc-L-allylglycine or Cbz-L-allylglycine wherein an amino group is protected with a protecting group, or a compound thereof wherein a carboxyl group is protected with a protecting group. Aforementioned Boc-L-allylglycine or Cbz-L-allylglycine can be easily produced using commercially avilable L-allylglycine according to the method described in PROTECTIVE GROUPS in ORGANIC SYNTHESIS (written by T.W. Green; P.G.M. Wuts). Furthermore, commercially available reagents of Boc-L-allylglycine or Cbz-L-allylglycine can also be preferably used.

In the step 1, the compound (2) is subjected to oxidative cleavage and, at the same time, it is subjected to a reductive amination reaction with azetidine-2-carboxylic acid. Oxidative cleavage is preferably performed, for example, using ozone, permanganate, RuCl₃ or OsO₄-NaIO₄, and oxidative cleavage with ozone is more preferred. Oxidation cleavage with ozone is preferably performed by, for example, blowing (bubbling) ozone gas into the solution in which the compound (2) is dissolved in a solvent. Examples of the solvent include an organic solvent such as methanol, dichloromethane, or ethyl acetate. Upon completion of oxidation cleavage with ozone, the solution turns blue when ozone is saturated in the solution. Therefore it is preferable to perform bubbling of ozone gas until the color of the solution turns blue. Bubbling of ozone gas is preferably performed at low temperature of about -100 to -50 °C. Ozone gas can be generated by ozone gas generator etc. In order to remove excessive ozone after bubbling of ozone gas, for example, an oxygen, nitrogen or argon gas is preferably bubbled into the solution until the blue color of the solution disappears.

The reductive amination reaction with azetidine-2-carboxylic acid performed simultaneously with oxidation cleavage is preferably performed in the presence of a reducing agent. The reducing agent is prefereably sodium cyanoborohydride or triacetoxy sodium borohydride. The pH in the reductive amination reaction is usually from about 4 to 7, and more preferably from about 6 to 7. Furthermore, the reductive amination reaction is usually performed while stirring under a cooling or warming, preferably at room temperature, for about 1 to 2 hours. The ratio of azetidine-2-carboxylic acid is preferably about 1 mol based on 1 mol of the compound (2) (the advantage of this synthesis is that high yield is obtained at a molar ratio 1:1). In addition, the ratio of the reducing agent to 1 mol of compound (2) is preferably within a range from about 1 to 2 mol, and more preferably from about 1.1 to 1.5 mol.

As L-azetidine-2-carboxylic acid, a commercially available reagent can be preferably used.

The compound represented by general formula (3) (hereinafter abbreviated to as a compound (3)) can be obtained by the above step 1.

The compound (3) is preferably a compound represented by general formula (3-1) or (3-2) (hereinafter abbreviated to as a compound (3-1) and a compound (3-2), respectively): 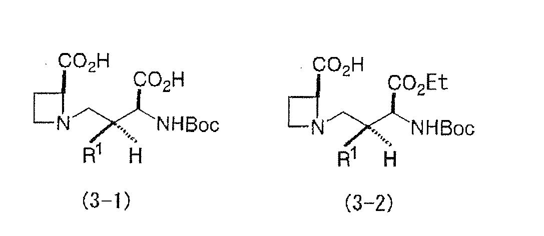 (wherein R¹ is as defined above).

### (2) Step 2

In the step 2, a compound represented by general formula (4) (hereinafter abbreviated to as a compound (4)) or their salts thereof can be obtained by protecting a carboxyl group of the compound (3) with a protecting group and eliminating the protecting group of an amino group. The reaction of protecting with the protecting group of the carboxyl group includes a dehydration condensation reaction with an alcohol. Examples of the alcohol used in the reaction include methanol, ethanol and tert-butanol. In the step 2, it is possible to perform the elimination reaction (hereinafter abbreviated to as deprotection) of the protecting group of an amino group by appropriately selecting a method using an acid or a base depending on the type of the protecting group, or the catalytic reduction method. The deprotection reaction is usually performed under cooling or heating and the reaction time is preferably from about 30 minutes to 24 hours, and more preferably from about 10 to 18 hours. Furthermore, the reaction temperature in elimination of a Boc group with an acid is preferably from about 0 °C to room temperature.

For example, when Boc is acting as the protecting group of an amino group, deprotection is preferably performed under strong acidic condition, for example, in trifluoroacetic acid or a hydrochloric acid-tetrahydrofuran solution. More specifically, the compound (3) is preferably reacted with a hydrochloric acid-ethanol solution. The reaction can be performed by, for example, stirring under ice cooling for about 30 minute to 5 hours, followed by stirring at room temperature for about 1 to 24 hours. The hydrochloric acid/ethanol solution (hereinafter abbreviated to as an ethanol hydrochloric acid) can be prepared by, for example, adding acetyl chloride to excessive ethanol. The volume of ethanol is, for example, from about 20 to 50 times, and more preferably from about 15 to 40 times as that of acetyl chloride. Alternatively, it can also be prepared by bubbling hydrochloric acid gas into ethanol. It is possible to determine the dissolution quantity of hydrochloric acid by comparing the weight of ethanol weighed beforehand with that of ethanol after the bubbling of the hydrochloric acid gas. After the reaction, the reaction mixture is subjected to, for example, vacuum concentration and the solvent is preferably distilled by azeotropic distillation by adding toluene. Furthermore, after the azeotropic distillation, the reaction mixture is preferably dried by sucking using a vacuum pump, thus obtaining a hydrochloride of a compound (4) in which a carboxyl group of the compound (3) is protected with a protecting group and, at the same time, the protecting group of an amino group is eliminated (general formula (4-1): 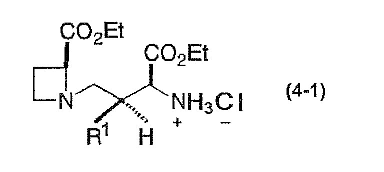 (wherein R¹ is as defined above).

Also, when the protecting group of an amino group is a benzyloxycarbonyl group (hereinafter abbreviated to Cbz), deprotection is preferably performed by, for example, a palladium catalyzed hydrogenation reaction and a Birch reaction.

### (3) Step 3

In the step 3, a reductive amination reaction of the aldehyde represented by general formula (5) (hereinafter abbreviated to as an aldehyde (5)) is performed using a compound (4) obtained in the step 2. It is possible to perform the reductive amination reaction in an organic solvent, similarly to the reductive amination reaction of the compound (2) in the presence of a reducing agent. Examples of the organic solvent include methanol, ethanol and dimethylformamide. Aldehyde (5) is easily produced according to the method described in, for example, Nishimaru, T. et al. Peptide Science 2006, 42, 263-266, or an analogue method thereof. The reaction mixture obtained from the reductive amination reaction contains compound represented by general formula (6) (hereinafter abbreviated to as a compound (6)). Isolation or purification of the compound (6) from the reaction mixture is preferred. The isolation or purification can be performed using conventionally known method, for example, extraction with an organic solvent such as ethyl acetate, chloroform or dichloromethane, and chromatography with silica gel as a carrier. These conventionally known methods can be performed either alone or in combinations.

### (4) Step 4

In the step 4, it is possible to perform the elimination (deprotection) reaction of the protecting group of a carboxyl group and the protecting group of a hydroxyl group or an amino group of compound (6) by appropriately selecting a method using an acid or a base depending on the type of protecting group, or the catalytic reduction method. In the case of acidic method, the acid varies depending on the type of protecting group and the other conditions, and examples thereof include an inorganic acid such as hydrochloric acid, hydrogen bromide, hydrogen fluoride, hydrogen iodide, methanesulfonic acid, p-toluenesulfonic acid, trifluoromethanesulfonic acid, sulfuric acid, or phosphoric acid; an organic acid such as formic acid, acetic acid, trifluoroacetic acid, or propionic acid; and acidic ion exchange resin. In the case of the method using a base, the base varies depending on the type of the protecting group and other conditions, and examples thereof include an inorganic base such as a hydroxide and a carbonate of an alkali metal such as sodium and potassium, as well as that of an alkaline earth metal such as calcium or magnesium; an organic base such as a metal alkoxide derivative, an organic amine derivative, or a quaternary ammonium salt; and a basic ion exchange resin. When a solvent is used in the case of the method using an acid or a base, for example, water, methanol, ethanol, ethyl acetate, chloroform, tetrahydrofuran, dioxane, pyridine, acetic acid, acetone, and methylene chloride can be used either alone or in combinations. Also, in the case of method using metal and an acid, water and acetone are often employed as the solvent. However, the acid itself can also be used as the solvent when the acid is in a liquid state. The reaction using an acid or a base is usually performed under cooling or heating, and the reaction time is from about 30 minutes to 20 hours, and more preferably from about 5 to 10 hours. Furthermore, the reaction temperature is preferably starting from about 0 °C and gradually increasing to room temperature. Deprotection of the protecting group of a carboxyl group and the protecting group of a hydroxyl group or an amino group of the compound (6) is preferably performed by the method using an acid, for example, hydrochloric acid, and more preferably by a method using about 4 to 6 N hydrochloric acid and 1N sodium hydroxide.

After the deprotection reaction, the compound represented by general formula (1) is isolated or purified from the reaction mixture. The isolation or purification can be performed using a conventionally known method, for example, liquid chromatography or recrystallization. Liquid chromatography includes ion exchange chromatography, partition chromatography, adsorption chromatography, and gel permeation chromatography, and such chromatography(s) can be performed either alone or in combinations.

In the step 1, it is possible to produce a compound as a raw material, in which R¹ of the compound (2) is a hydroxyl group, from a compound in which R¹ of the compound (2) is a hydrogen atom, by the following scheme: 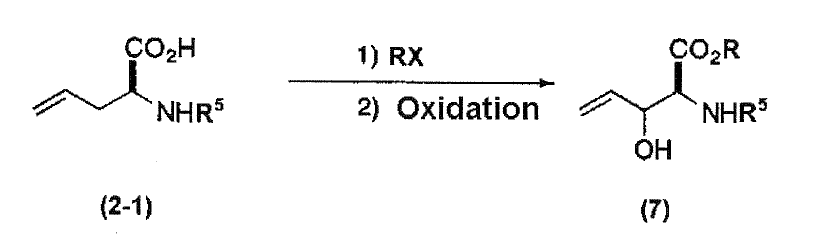 (wherein RX represents a halogenated alkyl, and R⁵ is as defined above).

First, the compound represented by general formula (2-1) (for example, Boc-L-allylglycine or Cbz-L-allylglycine; hereinafter abbreviated to as a compound (2-1)) is dissolved in a solvent and an alkyl halide is reacted with the compound (2-1) in the presence of base, thereby protecting a carboxyl group protected with a protecting group. Examples of the solvent include dimethylformamide, dimethylsulfoxide, and dioxane. The base is preferably potassium carbonate. Regarding the ratio of the compound (2-1) to the base such as potassium carbonate, the amount of the base is preferably from about 2 to 5 mol, and more preferably from 3 to 4 mol, based on 1 mol of the compound (2-1). When the alkyl group of the halogenated alkyl selected from the protecting group of a carboxyl group as exemplified above is employed, it is not required to perform deprotection and reprotection. Examples of the alkyl halide having such an alkyl group include an alkyl iodide compound such methyl iodide, ethyl iodide, n-propyl iodide, isopropyl iodide, n-butyl iodide, isobutyl iodide, or cyclohexyl iodide. Regarding the ratio of the compound (2-1) to the alkyl halide, the amount of the alkyl halide is preferably from about 1.1 to 1.5 mol based on 1 mol of the compound (2-1). The reaction temperature is preferably at room temperature under cooling to heating. The reaction time is usually from about 30 minutes to 8 hours, and preferably from about 1 to 3 hours. After the completion of the reaction, it is preferred that water or the like is added to the reaction mixture and the compound having a carboxyl group protected with a protecting group is preferably extracted with non-polar solvent (for example, ether, hexane, etc.) 1 to several times. The compound (2-1) having a carboxyl group protected with a protecting group can be obtained by collecting an extracted non-polar solvent, followed by desiccation using a desiccant such as magnesium sulfate, removal of the desiccant through filtration or the like and further concentration of the filtrate and distillation of the extracting solvent. The extracting solvent can be distilled off using a conventionally known method, for example, vacuum distillation.

Next, the compound thus obtained is preferably dissolved in a solvent and then oxidized. Examples of the solvent include an organic solvent such as 1,2-dichloroethane, methylene chloride, t-butanol, dioxane, toluene, and trichloroethane. Oxidation can be performed, in the presence of selenium dioxide, using an oxidizing agent, for example tert-butylperoxide or hydrogen peroxide. Example of the catalytic quantity of selenium dioxide is, for example, about 0.5 to 0.95 mol of selenium dioxide based on 1 mol of the compound (2-1). The quantity of the oxidizing agent is preferably within a range from about 2 to 5 mol, and more preferably from about 2.5 to 4 mol, based on 1 mol of the compound (2-1). Oxidation is preferably performed under heating at a preferred temperature of about 50 to 90 °C, and more preferably at about 60 to 70 °C. The reaction time in the oxidation is from about 1 to 24 hours, and preferably from about 5 to 10 hours. Posttreatment after the reaction can be conducted according to a conventional method. Namely, after the addition of an aqueous solution of sodium hydrogen carbonate or sodium hydroxide to the reaction solution, an organic solvent such as ethyl acetate, ether, dichloromethane or chloroform is added and extraction is performed 1 to several times. The extract is collected and dried with a desiccant such as magnesium sulfate. After drying, the desiccant is removed by filtration and the filtrate is concentrated by vacuum concentration and then the solvent is distilled off to obtain a product (7). The product (7) thus obtained is preferably purified by column chromatography or the like.

In this manner, it is possible to produce mugineic acids such as mugineic acid, 2'-deoxymugineic acid, 3-hydroxymugineic acid or 3-epihydroxymugineic acid and a precursor thereof such as nicotianamine or 2"-hydroxynicotianamine at high yield.

The present invention will now be described in more detail by way of examples, but the present invention is not limited thereto.

### Example 1: Production of Compound (3-3) from Boc-L-Allylglycine

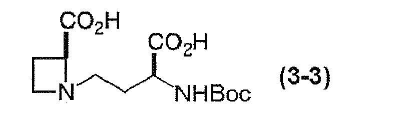

1 g (4.7 mmol) of Boc-L-allylglycine was dissolved in methanol (15 ml), followed by bubbling with ozone gas at -78 °C. After bubbling until color of the solution turned blue, oxygen was bubbled until the blue color disappeared. After returning to room temperature, 475 mg (4.7 mmol) of L-azetidine-2-carboxylic acid and 296 mg (4.7 mmol) of sodium cyanoborohydride were added, followed by stirring for one hour. After vacuum concentration of the reaction mixture, 1.3 g of compound (3-3) (yield: 90%) was obtained by short-pass silica gel chromatography with silica gel (after removing the residue of sodium cyanoborohydride and a small amount of byproduct derived from L-allylglycine with a mobile phase: a mixture solution of ethyl acetate:methanol= 9:1 (v/v), methanol solution was passed through).

### Example 2: Production of Compound (6-1) from Compound (3-3)

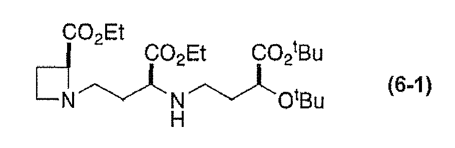

300 mg (1 mmol) of a compound (3-3) was dissolved in 15 ml of a 10% (v/v) ethanol hydrochloric acid (prepared from acetyl chloride and ethanol) at 0 °C, followed by stirring overnight at room temperature. The reaction mixture was subjected to vacuum concentration, dried by toluene azeotrope and then sucked using a vacuum pump until the mixture is dried. The hydrochloride thus produced was dissolved in 4 ml of methanol, and sequentially added with 250 mg (1.1 mmol) of aldehyde represented by the formula (5-1) (hereinafter abbreviated to as an aldehyde (5-1)): 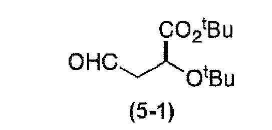 and 70 mg (1.1 mmol) of sodium cyanoborohydride, followed by stirring for about 2 hours. Saturated sodium hydrogen carbonate aqueous solution was added to the reaction mixture and the mixture was extracted 2 times with ethyl acetate. The extract was collected, dried over magnesium sulfate anhydride, filtered, and then vacuum-concentrated to obtain an oily substance. The resulting substance was purified by short-pass silica gel chromatography (mobile phase: ethyl acetate from hexane: ethyl acetate= 2:1 (v/v)) to obtain 372 mg of a compound (6-1) as a colorless oily substance (yield 79%).

### Example 3 : Production of 2'-Deoxymugineic Acid from Compound (6-1):

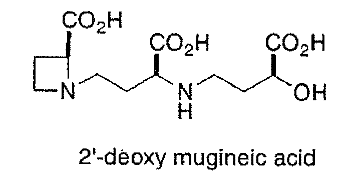

300 ml (0.6 mmol) of a compound (6-1) was added with 10 ml of 6 N hydrochloric acid, followed by stirring for about 15 hours. After vacuum concentration of the reaction mixture, 10 ml of an aqueous 1 N sodium hydroxide solution was added and, after stirring for 15 hours, the reaction mixture was neutralized with 1 N hydrochloric acid. After vacuum concentration of the reaction mixture, a hydrochloride of the crude 2'-deoxymugineic acid was obtained. This hydrochloride was dissolved in about 5 ml of water, treated with a Dowex 50Wx8 ion exchange resin and eluted with ammonia water to obtain a solution of an ammonium salt of 2'-deoxymugineic acid. This solution was subjected to vacuum desiccation to obtain 180 mg of crude 2'-deoxymugineic acid. The crude 2'-deoxymugineic acid was crystallized from water-methanol-ethanol to obtain 2'-deoxymugineic acid. The specific rotation and various spectra including ¹H NMR, ¹³C NMR, and MS of the resulting 2'-deoxymugineic acid agreed with those of the natural product (Nomoto, K. Chemia, (1981), 7, p249).

### Example 4 : Production of Compound (3-3) from Boc-L-Allylglycine

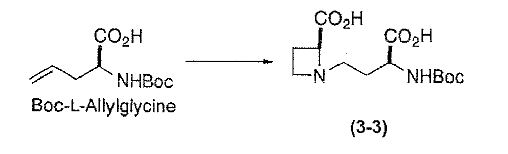

1.6g (7.4 mmol) of Boc-L-allylglycine was dissolved in methanol (25 ml), followed by bubbling with ozone gas at -78 °C. After bubbling until color of the solution turned blue, oxygen was bubbled until the blue color disappeared. After returning to room temperature, 752 mg (7.4 mmol) of L-azetidine-2-carboxylic acid and 470 mg (7.4 mmol) of sodium cyanoborohydride were added, followed by stirring for one hour. After vacuum concentration of the reaction mixture, 2.1 g of compound (3-3) (yield 93%) was obtained by short-pass silica gel chromatography with silica gel (after removing the residue of sodium cyanoborohydride and a small amount of byproduct derived from L-allylglycine with a mobile phase: a mixture solution of ethyl acetate: methanol = 5:1 (v/v), methanol solution was passed through).

### Example 5 : Production of Compound (3-4) from Boc-L-Allylglycine

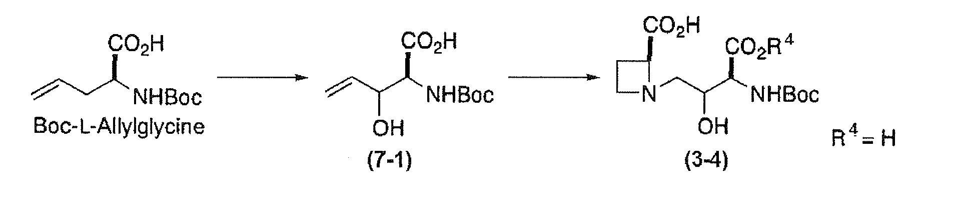

670 mg (3.1 mmol) of Boc-L-allylglycine was dissolved in 10 ml of dimethylformamide, and sequentially added with 1.3 g (9.1 mmol) of potassium carbonate and 0.32 ml (4.1 mmol) of ethyl iodide. The reaction mixture was stirred for 2 hours at room temperature, added with water, and extracted for 2 times with ether. The extract was collected, dried over magnesium sulfate, filtered, and then vacuum-concentrated to obtain a crude product of an ethyl ester derivative. The crude product was dissolved in 20 ml of 1,2-dichloroethane, added with 320 mg (2.9 mmol) of selenium dioxide and 2 ml (11.6 mmol) of 5.5 M tert-butylperoxide, followed by stirring at 70 °C for 8 hours. The reaction mixture was added with sodium hydrogen carbonate solution and extracted 2 times with ethyl acetate. The extract was collected, dried over magnesium sulfate, filtered, and then vacuum-concentrated to obtain a product (7-1). The resulting crude product was purified by silica gel column chromatography to obtain 400 mg of a product (7-1) as an oily substance (yield: 55%). The resulting product (7-1) was treated in the same manner as in Example 4 to obtain a compound (3-4) (yield: 80%).

### Example 6: Production of Compound (6-1) from compound (3-3)

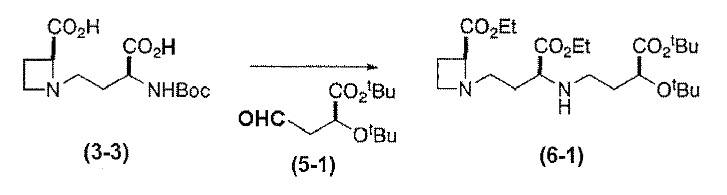

1.7 g (5.6 mmol) of a compound (3-3) was added with 140 ml of ethanol hydrochloric acid (prepared from 4 ml of acetyl chloride and 160 ml of ethanol) cooled at 0 °C, followed by stirring at 0 °C for 2 hours and further stirring at room temperature overnight. The reaction mixture was subjected to vacuum concentration, dried by toluene azeotrope and was sucked using a vacuum pump until it was dried. The hydrochloride thus produced was dissolved in 30 ml of methanol, and sequentially added with 1.3 g (5.6 mmol) of aldehyde (5-1) and 354 mg (5.6 mmol) of sodium cyanoborohydride, followed by stirring for about 5 hours. Saturated sodium hydrogen carbonate aqueous solution was added to the reaction mixture and was extracted 3 times with ethyl acetate. The extract was collected, dried over magnesium sulfate anhydride, filtered and then vacuum-concentrated to obtain an oily substance. This substance was purified by short-pass silica gel chromatography (mobile phase: ethyl acetate from hexane:ethyl acetate= 2:1 (v/v)) to obtain 1.8 g of a compound (6-1) as a colorless oily substance (yield: 68%).

### Example 7: Production of Compound (6-2) from Compound (3-4)

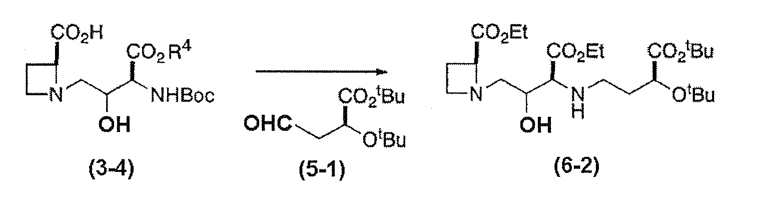

170 mg (0.49 mmol) of a compound (3-4) was added with 10.2 ml of ethanol hydrochloric acid (prepared from 0.2 ml of acetyl chloride and 10 ml of ethanol) cooled at 0 °C, followed by stirring at 0 °C for 2 hours and further stirring at room temperature overnight. The reaction mixture was subjected to vacuum concentration, dried by toluene azeotrope and sucked using a vacuum pump until it was dried. The hydrochloride thus produced was dissolved in 30 ml of methanol, and sequentially added with 113 mg (0.49 mmol) of aldehyde (5-1) and 31 mg (0.49 mmol) of sodium cyanoborohydride, followed by stirring for about 5 hours. The reaction mixture was subjected to vacuum concentration and then purified by short-pass silica gel chromatography (mobile phase: ethyl acetate from hexane:ethyl acetate= 2:1 (v/v)) to obtain 193 mg of a compound (6-2) as a colorless oily substance (yield: 81%).

### Example 8: One-Pot Production of Compound (6-1) from Boc-L-Allylglycine

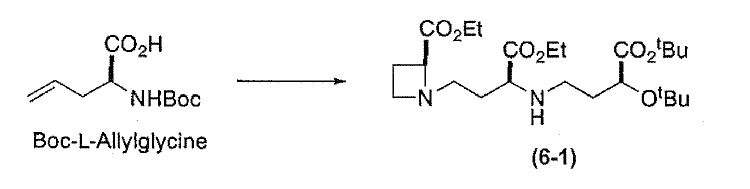

400 mg (1.9 mmol) of Boc-L-allylglycine was dissolved in methanol (15 ml), followed by bubbling with ozone at -78 °C. After bubbling until color of the solution turned blue, oxygen was bubbled until the blue color disappeared. 188 mg (1.9 mmol) of L-azetidine-2-carboxylic acid and 117 mg (1.9 mmol) of sodium cyanoborohydride were added, followed by stirring for 2 hours upon returning to room temperature. After vacuum concentration of the reaction mixture, 40 ml of ethanol hydrochloric acid (prepared from acetyl chloride 1.6 ml and ethanol 40 ml) cooled at 0 °C was added, followed by stirring at 0 °C for 2 hours and further stirring at room temperature overnight, and then dried by vacuum concentration, toluene azeotrope and a vacuum pump. The hydrochloride thus produced was dissolved in 20 ml of methanol, and sequentially added with 428 mg (1.9 mmol) of aldehyde (5-1) and 120 mg (1.9 mmol) of sodium cyanoborohydride, followed by stirring for about 5 hours. Saturated sodium hydrogen carbonate aqueous solution was added to the reaction mixture, followed by extraction 3 times with ethyl acetate. The extract was collected, dried over magnesium sulfate anhydride, filtered and then vacuum-concentrated to obtain an oily substance. This substance was purified by short-pass silica gel chromatography (mobile phase: ethyl acetate (added with 0.1% triethylamine) from hexane:ethyl acetate= 2:1 (v/v)) to obtain480 mg of a compound (6-1) as a colorless oily substance (yield: 55%).

### Example 9: Production of 2'-Deoxymugineic Acid from Compound (6-1)

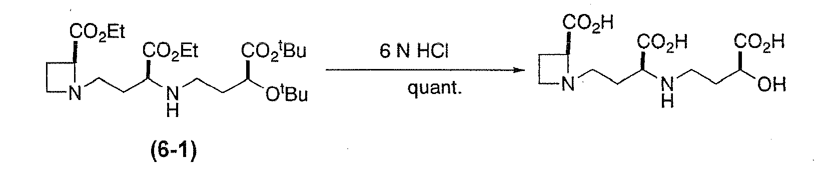

300 mg (0.6 mmol) of a compound (6-1) was added with 10 ml of 6 N hydrochloric acid at 0 °C, followed by stirring at room temperature for about 10 hours. After vacuum concentration of the reaction mixture, 15 ml of an aqueous 1 N sodium hydroxide solution was added, followed by stirring for 10 hours. After neutralization with 1 N hydrochloric acid, the reaction mixture was vacuum-concentrated to obtain a hydrochloride of the crude 2'-deoxymugineic acid. The resulting hydrochloride was dissolved in about 5 ml of water, treated with a Dowex 50Wx8 ion exchange resin and then eluted with ammonia water to obtain a solution of an ammonium salt of 2'-deoxymugineic acid. This solution was subjected to vacuum desiccation to obtain 180 mg of a crude 2'-deoxymugineic acid (yield: 100%). The crude 2'-deoxymugineic acid was crystallized from water-methanol-ethanol to obtain 120 mg of 2'-deoxymugineic acid (yield: 66%). The specific rotation and various spectra including ¹H NMR, ¹³C NMR, and MS of the resulting 2'-deoxymugineic acid agreed with those of the natural product (Nomoto, K. Chemia, (1981), 7, p249).

### Example 10: Production of Mugineic Acid from Compound (6-2)

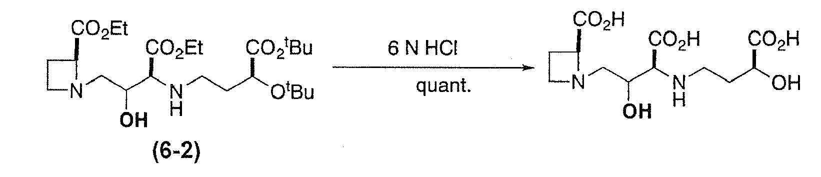

100 mg (0.31 mmol) of a compound (6-2) was added with 10 ml of 6N hydrochloric acid at 0 °C, followed by stirring at room temperature for about 2 hours. After vacuum concentration of the reaction mixture, 10 ml of an aqueous 1 N sodium hydroxide solution was added, followed by stirring for 10 hours. After neutralization with 1N hydrochloric acid, the reaction mixture was vacuum-concentrated to obtain a hydrochloride of the crude mugineic acid. This hydrochloride was dissolved in about 5 ml of water, treated with Dowex 50Wx8 ion exchange resin and then eluted with ammonia water to obtain a solution of an ammonium salt of mugineic acid. This solution was subjected to vacuum to obtain 99 mg of a crude mugineic acid. The powder thus obtained was dissolved in water 3 ml, and after purified with DIANION HP20 (eluted with water), it was once again subjected to freeze-drying to obtain 95 mg of mugineic acid (yield: 96%). The results of ¹H NMR and MS spectrum revealed that the resulting mugineic acid was a mixture of a natural form mugineic acid and a 2'-OH stereoisomer thereof.

### Example 11: Alternative method for Producing Mugineic Acid

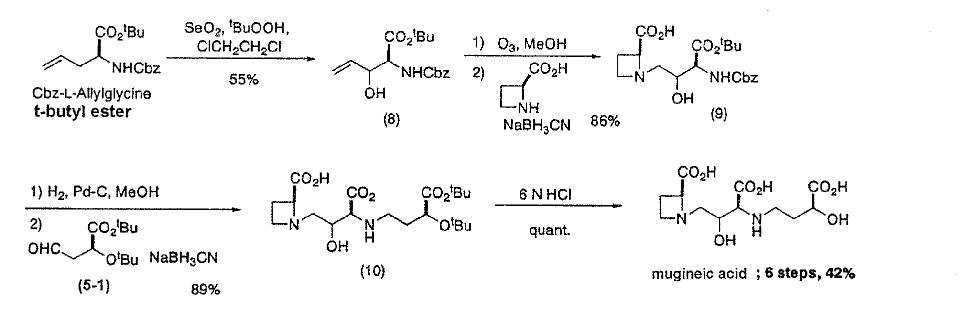

In accordance with Example 5, 1.4 g (4.6 mmol) of Cbz-L-allylglycine t-butyl ester was first converted into a compound (8) (yield: 55%), followed by converting 550 mg of a compound (8) into 580 mg (yield: 86%) of a compound (9). Subsequently compound (9) 450 mg (1.1 mmol) was dissolved in methanol 10 ml, added with 80 mg of 10% palladium carbon and was stirred for one hour in hydrogenous atmosphere. After diluting with methanol, it was aminated by Celite filtration and vacuum concentration. Next, the vacuum concentrate was dissolved in 15 ml of methanol, added with 66 µl of acetic acid, and then the pH was adjusted within a range from 4 to 6. Next, 254 mg (1.1 mmol) of aldehyde (5-1) and 70 mg (1.1 mmol) of sodium cyanoborohydride were sequentially added, followed by stirring for about 5 hours. The reaction mixture was subjected to vacuum concentration, and after the removal of sodium cyanoborohydride and the aldehyde residue by silica gel column chromatography using ethyl acetate/methanol (4:1 (v/v)) solution, the compound (10) was eluted with a chloroform/methanol (4:1 (v/v)) solution, followed by chloroform/methanol (2: 1 (v/v)) solution and further distillation of the eluate to obtain 480 mg of a compound (10) (yield: 89%).

200 mg (0.41 mmol) of the compound (10) thus obtained was dissolved in 6 N hydrochloric acid at 0 °C, stirred for about 2 hours and then vacuum-concentrated to obtain a hydrochloride of the crude mugineic acid. The hydrochloride was dissolved in about 5 ml of water, treated with Dowex 50Wx8 ion exchange resin and then eluted with ammonia water to obtain a solution of an ammonium salt of mugineic acid. This solution was subjected to vacuum desiccation to obtain a crude mugineic acid. The powder thus obtained was dissolved in 3 ml of water, and after purified with DIANION HP20 (eluted with water), it was once again subjected to freeze-drying to obtain 125 mg of mugineic acid (yield: 95%). The results of ¹H NMR and MS spectrum revealed that the resulting mugineic acid was a mixture of a natural form mugineic acid and a 2'-OH stereoisomer thereof.

### Example 12: Optical Resolution of Mugineic Acid

250 mg (0.51 mmol) of a compound (10) obtained in Example 11 was dissolved in 10 ml of an aqueous 20% acetonitrile solution (containing 0. 1% acetic acid), and was fractionated by liquid chromatography. Conditions of the liquid chromatography are as follows:
Column: CAPCELLPAK C18-UG80 (5µm) (Shiseido)
Elution rate: 9.999 ml/min
Elution condition: 35% CH₃CN-H₂O (1% acetic acid)
Elution time: Minor: 14.0 min; Major: 16.3 min
Injection volume: 200 µl each

Each fraction was collected, and the solvent was distilled off under reduced pressure. Each dried substance of the minor peak and major peak was dissolved in 6N HCl (20 ml), followed by stirring at room temperature for 5 hours. The solvent was distilled off under reduced pressure, and the resulting hydrochloride was carried on an ion exchange column (Dowex 50Wx8, 100-200 mesh, H⁺ form), eluted with an aqueous 5% NH₄OH solution, and then freeze-dried. The resulting powder was dissolved in water, and after purified with DIANION HP20 (eluted with water), it was once again subjected to freeze-drying. The yield of purified substance from the minor peak was 20 mg, whereas the yield of purified substance from the major peak was 81 mg. Measurement values of various physical properties of the purified substance from the minor peak and major peak are shown below.

### (Minor peak)

(a)²⁴_{D}-63.5°(c 0.31, H₂O);¹H NMR (400MHz, D₂O, pH=4.53 adjusted by the addition of 1N DCl)d 2.03 (1H, m), 2.19 (1H, m), 2.57 (1H, m), 2.72 (1H, m), 3.21 (1H, m), 3.29 (1H, m), 3.43 (1H, dd, J=2.7, 13.7 Hz), 3.56 (1H, dd, J=9.5, 13.7 Hz), 3.85 (1H, d, J=2.9 Hz), 4.03 (1H, app.q, J=9.7 Hz), 4.10 (1H, dt, J=4.2, 10.1 Hz), 4.17 (1H, dd, J=4.6, 7.3 Hz), 4.44 (1H, dt, J=9.3, 2.9 Hz), 4.88 (1H, t, J=9.5 Hz) ppm; ¹³C NMR (100MHz, D₂O, pH=4.53 adjusted by the addition of 1N DCl)d 24.6, 32.9, 47.9, 53.9, 59.0, 67.4 (2C), 70.6, 73.2, 171.8, 175.8, 182.3 ppm; IR (film, cm⁻¹) 3215, 3061, 2855, 1618, 1412, 1319, 1236, 1115, 1092, 976, 773; HRMS m/z calcd for C₁₂H₂₁N₂O₈⁺ (M+H)⁺: 321.1298, found: 321.1292.

### (Major peak)

(a)²⁴_{D}-48.8°(c 0.57, H₂O); ¹H NMR (400MHz, D₂O, pH=4.50 adjusted by the addition of 1N DCl)d 2.02 (1H, m), 2.16 (1H, m), 2.57 (1H, m), 2.70 (1H, m), 3.16 (1H, dt, J=12.7, 7.1 Hz), 3.30 (1H, dt, 12.7, 7.1 Hz), 3.41 (1H, dd, J=9.8, 13.2 Hz), 3.57 (1H, dd, J=2.7, 13.2 Hz), 3.63 (1H, d, J=8.3 Hz), 4.06 (1H, app.q, J=9.7 Hz), 4.09 (1H, m), 4.16 (1H, dd, J=4.4, 7.6 Hz), 4.22 (1H, ddd, J=2.7, 8.3, 9.8 Hz), 4.89 (1H, t, J=9.5 Hz); ¹³C NMR (100MHz, D₂O, pH=4.50 adjusted by the addition of 1N DCl)d 24.5, 32.7, 48.1, 54.4, 60.0, 67.4, 67.5, 69.9, 73.2, 172.4, 175.7, 182.1 ppm; IR (film, cm⁻¹) 3213, 3051, 2839, 1610, 1412, 1329, 1238, 1107, 934, 773; HRMS m/z calcd for C₁₂H₂₁N₂O₈⁺ (M+H)⁺: 321.1298, found: 321.1298.

Excluding the specific rotation, minor peak purified substance and major peak purified substance were almost identical. It was verified that the minor peak contains mugineic acid (S isomer of 2'-OH) of which configuration is the same as the natural form of the target substance whereas the major peak contains R isomer of 2'-OH. The ¹H NMR analysis revealed that a mol ratio of a S isomer to a R isomer is 1:3.

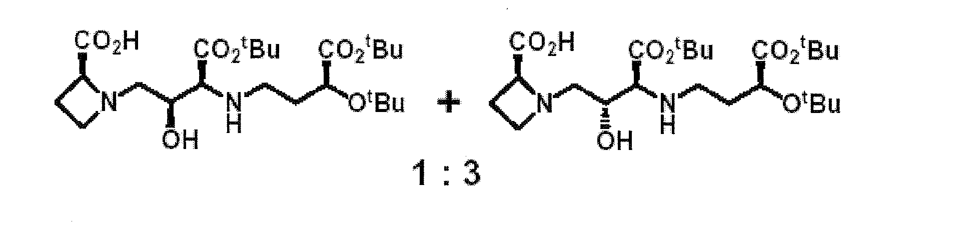

### Example 13: Verification of Activity of Synthetic Mugineic Acid

Concerning the S isomer (natural form) and R isomer of 2'-OH obtained in Example 12, 2'-deoxy isomer obtained in Example 9, and mugineic acid purified from barley by the method described in "Takagi, S. et al., Journal of Plant Nutrition, 1984, 7th volume, p469-477", it was verified that the intracellular uptake of iron complex thereof actually takes place via the mugineic acid-Fe complex transporter.

### (Production of African Clawed Frog Oocytes which express Mugineic Acid-Fe Complex Transporter)

According to the method described in "Murata, Y. et al., Plant Journal (Plant J), 2006, 46th volume, p.563-572.", the gene HvYS1 gene which codes the mugineic acid-Fe complex transporter of barley (Plant J. 2006, vol. 46, 563-572) was introduced into the oocytes of the African clawed frog. More specifically, HvYS1 cDNA (coding region of Sequence No. 1) was inserted into XbaI and BamHI sites of pSP64Poly (A) vector (Promega Corporation), and using this, cRNA was produced by mMESSAGE mMACHINE Kit of the Ambion Corporation.

Abdomen of the African clawed frog (purchased from Hamamatsu Seibutsu Kyozai Ltd. ) was dissected, and the oocytes (Xenopus Oocytes) were excised. Subsequently, Collagenase type IA (Sigma) was added so as it's concentration became 2 mg/mL, the oocyte was transferred to centrifuge tube containing OR-2 solution (82.5 mM NaCl, 2 mM KCl, 1 mM MgCl₂ and 5 mM HEPES), and after stirring for about 2 hours at room temperature, it was washed 3 times with OR-2 solution, and 3 times with ND-96 solution (96 mM NaCl, 2 mM KCl, 1 mM MgCl₂, 1.8 mM CaCl₂ and 5 mM HEPES). 50nL cRNA (50 µg/mL) were injected into the oocyte of the African clawed frog by digital type microdispenser (Drummond SCIENTIFIC). The oocytes were cultured at 17 °C for 48 to 72 hours in a ND-96 solution.

### (Preparation of Iron Complex of Mugineic Acids)

Each 30 mM of S isomer of 2'-OH, R isomer of 2'-OH, 2'-deoxy isomer and mugineic acid purified from barley was mixed with 30 mM of ferric chloride, left to stand at room temperature for 2 hours, and was diluted so as the iron complex concentration becomes 5 mM.

### (Comparison of Iron Uptake Activity)

The intracellular uptake of mugineic acid-Fe complex was measured using Oocyte clamp OC-725C (Warner Instrument) by the detection of electrochemical signal. More specifically, the oocyte which expressed the mugineic acid-Fe complex transporter HvYS1 was set to the chamber filled with ND-96 solution, 10 µl of a 5 mM substrate-Fe complex solution (final concentration 50 µM) was sprinkled on the oocyte and the electrical physiological activity was measured. The oocyte was plugged with 2 microelectrodes filled with 3M KCl, and with the mode in which the electric potential of the experimental tank was fixed at 0 V, the current value changing at a fixed electrical potential, 60 mV, was measured. The data was recorded with the PowerLab data-recording device (Chart 4 software).

The results are shown in Fig. 1. The size of the current value (electrical physiological activity) measured for S isomer of 2'-OH, R isomer of 2'-OH and 2'-deoxy isomer was showed as a relative value when the size of the electrical signal (electrical physiological activity) measured for the natural mugineic acid was set to 100%. Each of S isomer of 2'-OH, R isomer of 2'-OH and 2'-deoxy isomer was similar to the natural mugineic acid. Similar to the mugineic acid obtained by one pot synthesis, these results suggest that both the mixed configuration of 2'-OH of the synthesized mugineic acid, and the 2'-deoxy isomer are useful for the transport of iron complex into the plant.

### INDUSTRIAL APPLICABILITY

The method of the present invention is useful in large quantity production of mugineic acids utilizable as a chelating agent replacing EDTA at low cost. Also, mugineic acids produced by the method of the present invention can be utilized in research concerning iron transport mechanism of plants, as well as in various fields such as health foods, cosmetics and fertilizers.

## Claims

1. A method for producing mugineic acids represented by the following general formula (1): 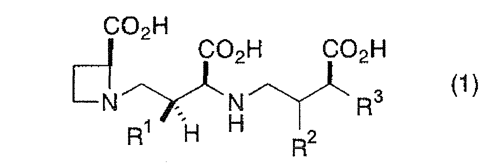 (wherein R¹ represents a hydrogen atom or a hydroxyl group, R² represents a hydrogen atom or a hydroxyl group, and R₃ represents a hydroxyl group or an amino group), which comprises:
a step 1 of simultaneously performing oxidative cleavage of a vinyl group of a compound represented by the following general formula (2): 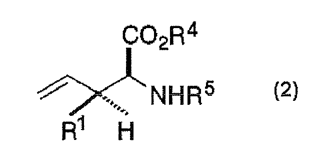 (wherein R¹ is as defined above, R⁴ represents a hydrogen atom or a protecting group of a carboxyl group, and R⁵ represents a protecting group of an amino group), and a reductive amination reaction with azetidine-2-carboxylic acid to obtain a compound represented by the following general formula (3): 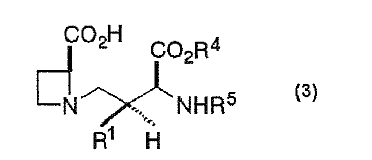 (wherein R¹, R⁴ and R⁵ are as defined above);
a step 2 of protecting a carboxyl group of the compound represented by the general formula (3) with a protecting group and removing the protecting group of the amino group to obtain a compound represented by the following general formula (4): 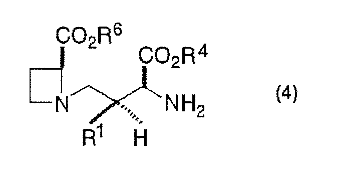 (wherein R¹ and R⁴ are as defined above, and R⁶ represents a protecting group of a carboxyl group) or a salt thereof;
a step 3 of subjecting an aldehyde represented by the following general formula (5): 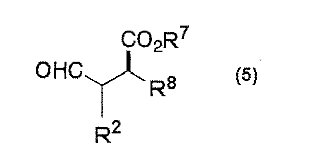 (wherein R² is as defined above, R⁷ represents a protecting group of a carboxyl group, and R⁸ represents -OR⁹ (wherein R⁹ represents a protecting group of a hydroxyl group) or -NHR¹⁰ (wherein R¹⁰ represents a protecting group of an amino group)) to an reductive amination reaction with the compound represented by the above general formula (4) to obtain a compound represented by the following general formula (6): 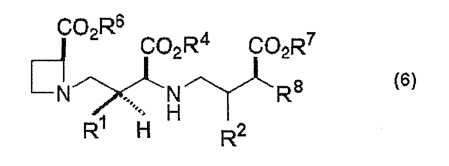 (wherein R¹, R², R⁴, R⁶ , R⁷ and R⁸ are as defined above); and
a step 4 of removing the protecting group of the carboxyl group and the protecting group of the hydroxyl group or the amino group of the compound represented by the above general formula (6).

2. A compound represented by the following general formula (3-1): 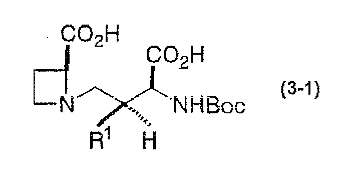 (wherein R¹ represents a hydrogen atom or a hydroxyl group, and Boc represents a tert-butoxycarbonyl group).

3. A compound represented by the following general formula (4-1): 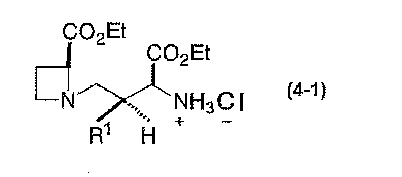 (wherein R¹ represents a hydrogen atom or a hydroxyl group, and Et represents an ethyl group).
